# EUROPEAN PATENT APPLICATION

(11) **EP 3 819 388 A1**
(43) Date of publication of application: **12.05.2021**
(21) Application number: 19382982.7
(22) Date of filing: 11.11.2019
(51) Int. Cl.: C12Q 1/6886

(54) **IN VITRO METHOD FOR THE PROGNOSIS OF ANAL SQUAMOUS CELL CARCINOMA**

(71) Applicant: Grupo Español Multidisciplinar en Cáncer Digestivo (GEMCAD), 08024 Barcelona (ES); Biomedica Molecular Medicine SL (BMM), 28049 Madrid (ES)
(72) Inventor: FELIU BATTLE, Jaime, 28046 Madrid (ES); GHANEM CAÑETE, Ismael, 28046 Madrid (ES); GÁMEZ POZO, Angelo, 28049 Madrid (ES); FRESNO VARA, Juan Ángel, 28046 Madrid (ES); TRILLA FUERTES, Lucía, 28049 Madrid (ES); FERNÁNDEZ MARTOS, Carlos, 08024 Barcelona (ES)
(74) Representative: Hoffmann Eitle

(57) **Abstract**

*In vitro* method for the prognosis of anal squamous cell carcinoma. The present invention refers to an *in vitro* method for the prognosis of anal squamous cell carcinoma (ASCC), which comprises determining the presence or the absence of a gene duplication located in CYP2D locus, preferably a duplication of the genes CYP2D6, CYP2D7P and/or CYP2D8P.

## Description

### FIELD OF THE INVENTION

The present invention pertains to the medial field. Particularly, the present invention refers to an *in vitro* method for the prognosis of anal squamous cell carcinoma (ASCC), which comprises determining the presence or the absence of a gene duplication located in CYP2D locus, preferably a duplication of the gene CYP2D6, CYP2D7P and/or CYP2D8P.

### STATE OF THE ART

ASCC is an infrequent tumor. In 2019, it has been estimated that 8,300 new cases will appear in the United States, representing 2.7% of all gastrointestinal cancers.

The standard treatment is a combination of 5-fluorouracil (5FU) with mitomycin C or cisplatin, concomitantly with radiotherapy since 1970s. This treatment has demonstrated its efficacy in early-stage tumors. However, in T3-T4 or N1 tumors, disease-free survival (DFS) ranges between 40-70%. In addition, around 40-70% of patients with ASCC tumors in advanced stages are going to suffer a relapse. For these reasons, it is necessary the molecular characterization of this disease in order to establish possible targeted therapies and a personalized follow-up.

Therefore, there is still an unmet medical need of finding reliable tools which can be used to carry out an effective prognosis of the patients suffering from ASCC. These new tools would improve the management of these patients and would help the clinicians to adapt the treatment to each specific patient.

In fact, by using high-throughput sequencing techniques, it is possible the study multiple genetic alterations in clinical samples. Whole-exome sequencing (WES) is now incorporating into the clinical practice. Additionally, several works have associated genetic alterations with prognosis in cancer.

The present invention is indeed focused on solving the above cited problem and it is directed to an *in vitro* method for the prognosis of ASCC. For this purpose, a discovery and a validation cohort were analyzed by whole-exome sequencing, finally identifying reliable biomarkers which can be used for the prognosis of ASCC.

### DESCRIPTION OF THE INVENTION

### Brief description of the invention

The present invention refers to an *in vitro* method for the prognosis of ASCC, which comprises determining the presence or the absence of a gene duplication located in CYP2D locus, preferably a duplication of the gene CYP2D6 (Ensembl: ENSG00000100197), CYP2D7P Ensembl: ENSG00000205702 and/or CYP2D8P (Ensembl: ENSG00000226450).

Precisely, twenty-nine genes were analysed in the present invention whose duplication was significantly related with disease-free survival (DFS) in ASCC patients. Of those genes, it was remarkable those duplications located in CYP2D locus, which involved CYP2D6, CYP2D7P or CYP2D8P genes among others. When the length of these CYP2D duplications was analysed, it was determined that the minimum unit always involved CYP2D6 gene. For this reason, the present invention refers to the use of a gene duplication located in CYP2D locus as a prognosis biomarker for ASCC, particularly the duplication of the genes CYP2D6, CYP2D7P or CYP2D8P, more preferably the duplication of the gene CYP2D6 which was studied in depth in the present invention because, as explained above, it represent the minimum unit in CYP2D locus which can be used a prognosis biomarker for ASCC.

So, in a preferred embodiment, the present invention reveals that the number of copies of the gene CYP2D6 is related to disease-free survival (DFS) in ASCC patients. Particularly, the duplication of the gene CYP2D6 can be used as a prognostic biomarker, which defines what patients are going to relapse, and therefore help the clinicians in the making-decision process. Those patients that are going to relapse could be redirected to a clinical trial.

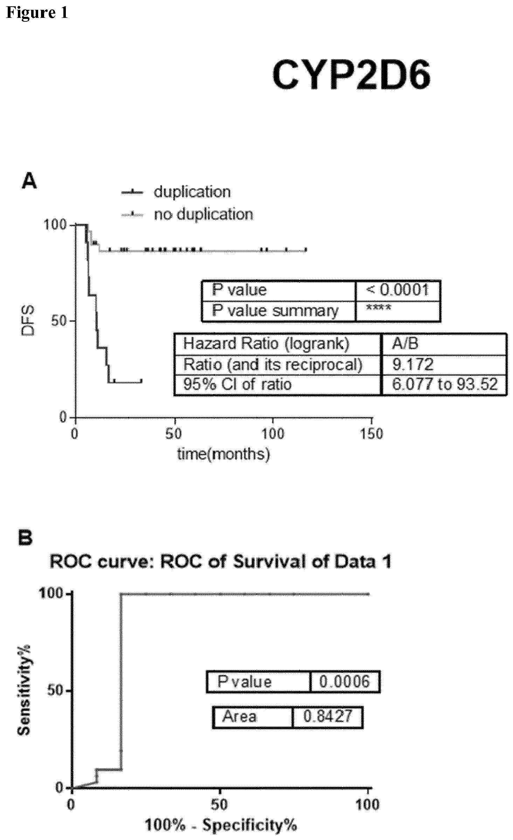

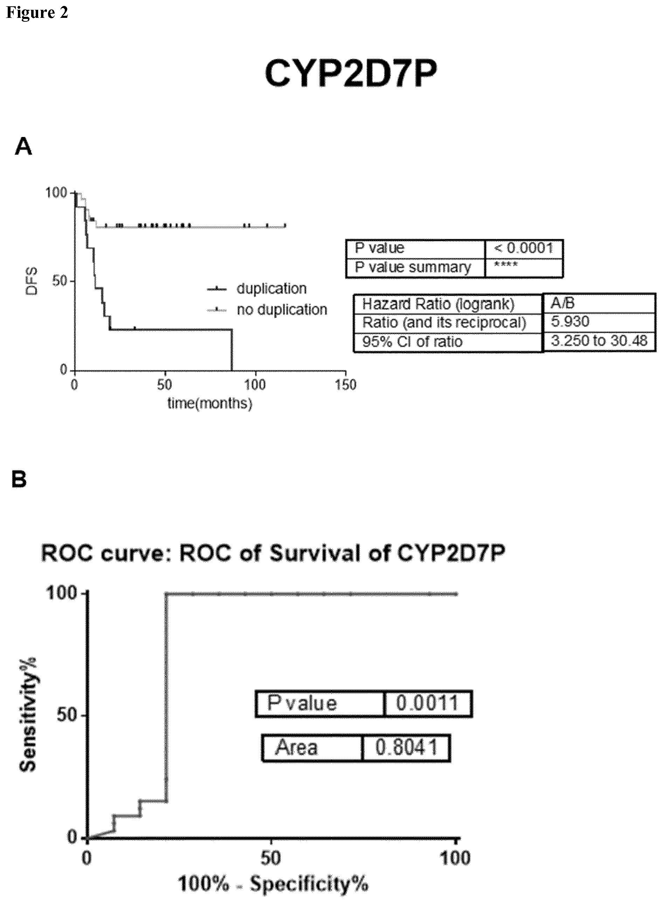

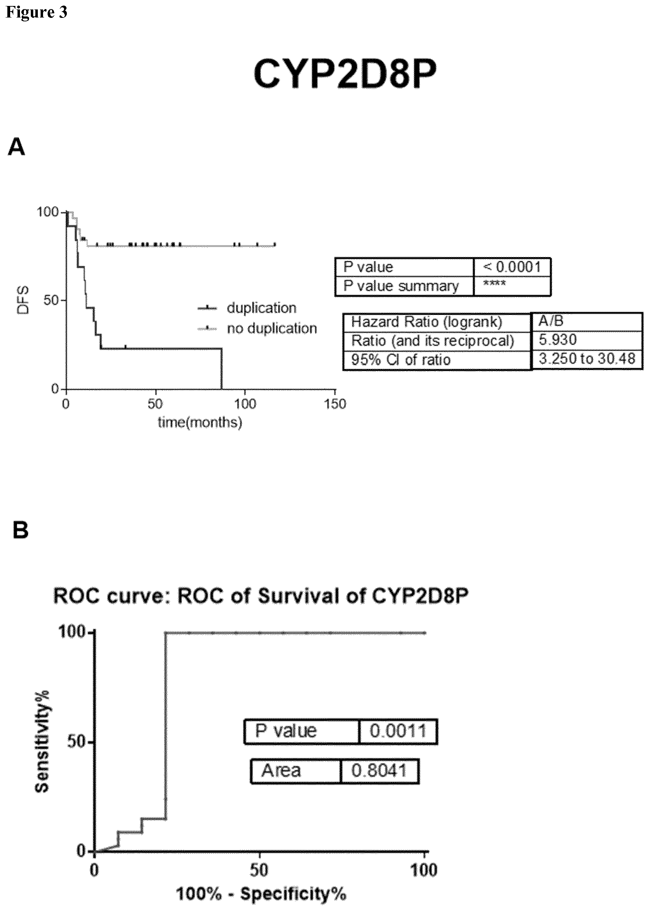

In this regard, please refer to the **Figure 1****,** **Figure 2** and **Figure 3** wherein DFS curves according CYP2D6, CYP2D7P or CYP2D8P status, and ROC curves for CYP2D6, CYP2D7P or CYP2D8P prediction are represented. Such as it can be seen in the figures, the AUCs for CYP2D6, CYP2D7P and CYP2D8P are, respectively: 0.84, 0.80 and 0.80. This means that the presence of duplications in any gene located in CYP2D locus, preferably in any of the genes CYP2D6, CYP2D7P and CYP2D8P can be used for the prognosis of ASCC.

So, the first embodiment of the present invention refers to an *in vitro* method for the prognosis of ASCC (hereinafter the method of the invention), which comprises determining the presence or the absence of a gene duplication located in CYP2D locus, in a biological sample obtained from the patient, wherein the presence of a gene duplication located in CYP2D locus is an indication that the patient has a poor prognosis and the absence of a gene duplication located in CYP2D locus is an indication that the patient has a good prognosis.

In a preferred embodiment, the method of the invention comprises determining the presence or the absence of a duplication of the gene CYP2D6, CYP2D7P and/or CYP2D8P (which are located in CYP2D locus), in a biological sample obtained from the patient, wherein the presence of a duplication of the gene CYP2D6, CYP2D7P and/or CYP2D8P is an indication that the patient has a poor prognosis and the absence of a duplication of the gene CYP2D6, CYP2D7P and/or CYP2D8P is an indication that the patient has a good prognosis.

In a preferred embodiment, the biological sample is tissue, plasma, blood or serum.

In a preferred embodiment, the prognosis is confirmed by an image technique or the patient is monitored by using an image technique.

The second embodiment of the present invention refers to a kit adapted for the prognosis of anal squamous cell carcinoma (ASCC) which comprises:
a. Tools or reagents for obtaining a sample from the patient, and
b. Tools or reagents for determining the presence or absence of a duplication of the gene located in CYP2D locus, such as Multiplex Ligation-dependent probe amplification (MPLA) or TaqMan Copy Number Assays and qPCR, or Capture-based sequencing.

In a preferred embodiment the kit comprises tools or reagents for determining the presence or absence of a duplication of the gene CYP2D6, CYP2D7P and/or CYP2D8P.

In a preferred embodiment, the sample is tissue, plasma, blood or serum.

The third embodiment of the present invention refers to the *in vitro* use of a gene located in CYP2D locus for the prognosis of ASCC.

In a preferred embodiment, the present invention refers to the *in vitro* use of the gene CYP2D6, CYP2D7P and/or CYP2D8P for the prognosis of ASCC.

The fourth embodiment of the present invention refers to the use of the above defined kit for the prognosis of ASCC.

The fifth embodiment of the present invention refers to a method for treating ASCC characterized in that the method comprises a first stage wherein the prognosis of the patient is identified by using the above defied method of the invention. Alternatively, the present invention refers to an anticancer therapy for use in a method for the treatment of ASCC, characterized in that the method comprises a first stage wherein the prognosis of the patient is identified by using the above defied method of the invention. In a preferred embodiment the treatment is chemotherapy, preferably in combination with radiotherapy. In a still preferred embodiment the treatment a combination of 5-fluorouracil (5FU) with mitomycin C or cisplatin, along with radiotherapy.

For the purpose of the present invention the following terms are defined:
- The term "duplication" or "amplification" refers to a type of mutation that involves the production of one or more copies of a gene or region of a chromosome. It can be defined as any duplication of a region of DNA that contains a gene.
- The term "comprising" it is meant including, but not limited to, whatever follows the word "comprising". Thus, use of the term "comprising" indicates that the listed elements are required or mandatory, but that other elements are optional and may or may not be present.
- By "consisting of' is meant including, and limited to, whatever follows the phrase "consisting of'. Thus, the phrase "consisting of' indicates that the listed elements are required or mandatory, and that no other elements may be present.

### Brief description of the figures

**Figure 1****. A)** DFS curves according CYP2D6 status. **B)** ROC curve for CYP2D6 prediction with an AUC of 0.84.
**Figure 2****. A)** DFS curves according CYP2D7P status. **B)** ROC curve for CYP2D7P prediction with an AUC of 0.80.
**Figure 3****. A)** DFS curves according CYP2D8P status. **B)** ROC curve for CYP2D8P prediction with an AUC of 0.80.

### Detailed description of the invention

### Example 1. Material and methods

### Example 1.1. Patient characteristics

Forty-six formalin-fixed paraffin-embedded (FFPE) samples from patients diagnosed with localized ASCC were used in this study. Tumor samples were reviewed by a pathologist and all the samples contained at least 70% of tumor cells. This study was approved by the Ethical Committee of Hospital Universitario La Paz and informed consent was obtained for all the participants in the study. The inclusion criteria were: have a histologically-confirmed ASCC; be 18 years or older; have an Eastern Cooperative Oncology Group performance status score (ECOG-PS) from 0 to 2; have not received prior radiotherapy or chemotherapy for this malignancy, and no presence of distant metastasis at diagnosis. Human papilloma virus (HPV) infection was determined by CLART® HPV2 (Genomica).

### Example 1.2. DNA isolation.

One 10 mm slide from each FFPE sample was deparaffinized and DNA was isolated with GeneRead DNA FFPE Kit (Qiagen), following manufacturer's instructions. One eluted, DNA was stored at -80°C until use.

### Example 1.3. Library preparation, exome capture and Illumina sequencing.

For the whole-exome sequencing experiments, purified DNA was quantified by Picogreen and mean size was measured by gel electrophoresis. Genomic DNA was fragmented by mechanical means (Bioruptor) to a mean size of around 200 bp. DNA tests were repaired, phosphorylated, A-tailed and ligated to specific adaptors. Then, PCR-mediated labeling with Illumina-specific sequences and sample specific barcodes (Kapa DNA library generation kit) was done.

Exome capture was done using VCRome system (capture size 37 Mb, Roche Nimblegen) under a multiplexing of 8 samples per capture per reaction. After the capture, libraries were cleaned, quantified and titrated by Real Time PCR before sequencing. Sequencing was performed with coverage of 4.5 Gb per sample using a Illumina NextSeq NS500 (Illumina Inc.).

### Example 1.4. Bioinformatics processing of exome sequencing data.

For quality verification, FASTQC was used. Using Cutadapt, adaptors were removed. Then, FASTQ files were filtered by quality using Prinseq. Both tools are integrated in GPRO Suite (Biotechvana SL). Sequence alignment was done using the human genome h19 as reference and BWA, Samtools, and Picard Tools. Variant calling was performed using GATK package and a panel of normal samples (PON) to standardize the copy proportions and remove sample noise. Then, Variant Effect Predictor (VEP) was used to annotate the Copy Number Variants (CNVs).

### Example 1.5. Prognostic signature.

Duplications associated with DFS were determined using BRB Array Tools (19). A p-value under 0.001 was considered as statistically significant in order to correct the multitesting.

### Example 1.6. Validation of CYP2D locus duplication prognostic value.

To validate the results, an independent cohort of ASCC patients has been used. The validation can be performed by using TaqMan qPCR Copy Number Assay (Thermo Fisher). It is possible to use this technique in both paraffin and blood samples. Alternatively, the validation can be done by using MLPA (MRC Holland).

CYP2D6, CYP2D7P and CYP2D8P amplifications could be measured in blood samples. In this case, this test has another advantage: the possibility to use a less invasive test like a blood test to determine if a patient is going to relapse.

### Example 1.7. Statistical analyses.

Statistical analyses were done using GraphPad Prism v6.

### Example 2. Results.

### Example 2.1. Patient cohort.

Forty-six FFPE samples from patients diagnosed with localized ASCC were used in this study. Twenty-seven were from VITAL clinical trial (GEMCAD-09-02, NCT01285778). These patients received panitumumab, 5FU and mitomycin C, concomitantly with radiotherapy. The remaining eighteen patients were recruited from the daily clinical practice of Hospital Universitario La Paz and Hospital Clinic of Barcelona. Of those 18 patients, fourteen were treated with 5FU and mitomycin or 5FU and cisplatin, concomitantly with radiotherapy. Three patients were treated with surgery alone and one patient was treated with radiotherapy alone. These four patients were excluded for the survival analyses.

### Example 2.2. Whole-exome sequencing experiments.

The mean coverage of whole-exome sequencing (WES) experiments was > 42.6x, except for one sample with a coverage of 3.57x that was excluded for the subsequent analyses. The rest of the samples displayed a mapping efficiency of 90-98%, with the exception of a sample (75.4%). The human exome includes > 195,000 exonic regions, out of which just 23,021 (11.21%) were not mapped in any sample.

### Example 2.3. CYP2D locus amplifications had prognostic value in ASCC patients.

Twenty-nine genes in which the presence of duplications was significantly related with DFS in ASCC were determined. Of those genes, it was remarkable those duplications located in CYP2D locus, involved CYP2D6, CYP2D7P and CYP2D8P genes among others. Studying the length of these CYP2D duplications, the minimum unit always involved *CYP2D6* gene. For this reason, CYP2D6 duplication was preferably studied in our cohort.

Patients that presented duplication in CYP2D6, CYP2D7P or CYP2D8P genes presented worse prognosis than patients without duplication in these genes (see **Figure 1****,** **2** **and** **3**).

## Claims

1. *In vitro* method for the prognosis of anal squamous cell carcinoma (ASCC), which comprises determining the presence or the absence of a gene duplication located in CYP2D locus, in a biological sample obtained from the patient, wherein the presence of a gene duplication located in CYP2D locus is an indication that the patient has a poor prognosis and the absence of a gene duplication located in CYP2D locus is an indication that the patient has a good prognosis.

2. *In vitro* method, according to claim 1, which comprises determining the presence or the absence of a duplication of the gene CYP2D6, CYP2D7P and/or CYP2D8P, in a biological sample obtained from the patient, wherein the presence of a duplication of the gene CYP2D6, CYP2D7P and/or CYP2D8P is an indication that the patient has a poor prognosis and the absence of a duplication of the gene CYP2D6, CYP2D7P and/or CYP2D8P is an indication that the patient has a good prognosis.

3. *In vitro* method, according to any of the previous claims, wherein the biological sample is tissue, plasma, blood or serum.

4. *In vitro* method, according to any of the previous claims, wherein the prognosis is confirmed by an image technique.

5. Kit adapted for the prognosis of anal squamous cell carcinoma (ASCC) which comprises:
a. Tools or reagents for obtaining a sample from the patient, and
b. Tools or reagents for determining the presence or absence of a duplication of the gene located in CYP2D locus.

6. Kit, according to claim 5, which comprises tools or reagents for determining the presence or absence of a duplication of the gene CYP2D6, CYP2D7P and/or CYP2D8P.

7. Kit, according to any of the claims 5 or 6, wherein the sample is tissue, plasma, blood or serum.

8. *In vitro* use of a gene located in CYP2D locus for the prognosis of anal squamous cell carcinoma (ASCC).

9. *In vitro* use, according to claim 8, of the gene CYP2D6, CYP2D7P and/or CYP2D8P for the prognosis of anal squamous cell carcinoma (ASCC).

10. Use of the kit of claims 5 to 7 for the prognosis of anal squamous cell carcinoma (ASCC).

11. Anticancer therapy for use in a method for the treatment of anal squamous cell carcinoma (ASCC), **characterized in that** the method comprises a first stage wherein a bad prognosis of the patient is identified according to the claims 1 to 3.

12. Anticancer therapy for use, according to claim 8, wherein the therapy comprises a combination of 5-fluorouracil (5FU) with mitomycin C or cisplatin, along with radiotherapy.
